Europäisches Patentamt

European Patent Office

Office européen des brevets

(11) Veröffentlichungsnummer: **0 272 645 B1**

(12) **EUROPÄISCHE PATENTSCHRIFT**

(45) Veröffentlichungstag der Patentschrift: **04.11.92**

(21) Anmeldenummer: **87118822.3**

(22) Anmeldetag: **18.12.87**

(51) Int. Cl.⁵: **G01N 33/34**, G01N 21/35, G01N 23/16

(54) Verfahren zur selektiven Füllstoffmessung an laufenden Materialbahnen, insbesondere Papierbahnen.

(30) Priorität: **20.12.86 DE 3643764**

(43) Veröffentlichungstag der Anmeldung:
**29.06.88 Patentblatt 88/26**

(45) Bekanntmachung des Hinweises auf die Patenterteilung:
**04.11.92 Patentblatt 92/45**

(84) Benannte Vertragsstaaten:
**DE ES FR GB IT NL SE**

(56) Entgegenhaltungen:
**EP-A- 0 112 079**
**WO-A-85/03351**
**GB-A- 1 115 904**
**GB-A- 2 044 443**

**Fachlexikon ABC Physik 1982, Seiten 1328-1331**

(73) Patentinhaber: **Ahlstrom Automation GmbH & Co. KG**
**Wilhelm-Leuschner-Strasse 12**
**W-5450 Neuwied 1(DE)**

(72) Erfinder: **Mercer, Peter George**
**Vulkanstr. 1**
**W-5475 Burgbrohl(DE)**

(74) Vertreter: **Lippert, Hans, Dipl.-Ing. et al**
**Reichel und Reichel Parkstrasse 13**
**W-6000 Frankturt (Main) 1(DE)**

EP 0 272 645 B1

## Beschreibung

Die Erfindung bezieht sich auf ein Verfahren zur Messung des Flächengewichts von Stoffen in einer laufenden Papierbahn aus einer Materialmischung mit Cellulose als Trägerstoff, Kaolin und/oder Talkum als erste Füllstoffkomponente, Satinweiß, Calciumcarbonat, Titanoxid oder Bariumsulfat als zweite Füllstoffkomponente und Wasser als weiterer Stoff.

Es ist bekannt, durch Anwendung einer Beta-Strahlung das Flächengewicht einer laufenden Papierbahn zu messen. Ferner ist es bekannt, durch gleichzeitige Anwendung einer Beta-Strahlung und einer charakteristischen Röntgenstrahlung an laufenden Papierbahnen den gesamten Asche- oder Füllstoffgehalt zu messen, allerdings unter der Voraussetzung, daß entweder in der Papierbahn nur ein einziger Füllstoff, nämlich Kaolin, Talkum oder eine Füllstoffkomponente mit reinem $CaCO_3$ enthalten ist oder daß im Falle eines Füllstoffkomponentengemische, nämlich Kaolin, Talkum, $CaCO_3$ und/oder $TiO_2$, die Füllstoffkomponentenrezeptur, d.h. das Verhältnis zwischen den einzelnen Füllstoffkomponenten, bekannt ist.

Bei diesem bekannten Meßverfahren wird als Quelle für die charakteristische Röntgenstrahlung eine natürliche radioaktive Fe-55-Strahlungsquelle verwendet, die eine Quantenstrahlung mit einer monochromatischen Energie von 5,9 keV emittiert. Das bekannte Füllstoffmeßverfahren nutzt den Umstand aus, daß bei Verwendung des monoenergetischen Meßstrahls der Fe-55-Strahlungsquelle ein streng exponentielles Absorptionsgesetz mit wohl definierten Absorptionskoeffizienten gilt. Dies bedeutet, daß sich alle Strahlungsextinktionsbeiträge der verschiedenen Bestandteile der untersuchten Materialbahn mathematisch zu einem resultierenden Extinktionssignal aufaddieren.

Weiterhin ist ein die Röntgenfluoreszenzanalyse ausnutzendes Meßverfahren zur Messung des Füllstoffgehalts in Papierbahnen bekannt. Gemessen werden kann hiermit ein Füllstoffkomponentengemisch, das Mg, Ca, Al und Ti enthält. Ausgenutzt wird dabei der Umstand, daß jedes der Elemente Mg, Ca, Al und Ti eine spezifische Fluoreszenzwellenlänge aufweist. Der Gehalt jedes der Elemente in der untersuchten Papierbahn wird dadurch gewonnen, daß die elementspezifische Fluoreszenz pro Zeiteinheit gemessen wird. Diese analytische Methode, in Geräten für die labormäßige Probenanalyse heute standardmäßig eingesetzt, hat sich wegen schwerwiegender apparativer Probleme bis heute im on-line Einsatz nicht durchgesetzt. Die Probleme sind im wesentlichen: Temperaturempfindlichkeit des Stintillationszählers für den Nachweis der Fluoreszenzstrahlung; Anwendung der Remissionsgeometrie mit dem Problem des nicht konstanten Abstandes der Probe zum Strahler und Detektor bei bewegten Bahnen.

Andererseits ist es aus der DE-PS 29 10 673 bekannt, den absoluten Gehalt oder das Flächengewicht eines Stoffes in einer laufenden Materialbahn unter Anwendung von Infrarotstrahlung und Beta-Strahlung mit hoher Genauigkeit zu messen, sofern sowohl der zu messende Stoff als auch ein die Materialbahn im wesentlichen bildender weiterer Stoff jeweils eine ausgeprägte Absorptionsbande im Infrarotstrahlungsbereich haben und die Stoffe des Material gemischt vorliegen, d.h. eine Materialmischung bilden. Dieses bekannte Meßverfahren wird insbesondere angewendet zur Ermittlung des absoluten Gehalts von Wasser in Papierbahnen mit Cellulose als Hauptsubstanz. Die Messung im Infrarotstrahlungsbereich erfolgt mit Hilfe einer vom Wasser absorbierten Meßwellenlänge, einer von der Cellulose absorbierten Meßwellenlänge und einer sowohl vom Wasser als auch von der Cellulose im wesentlichen nicht absorbierten Vergleichswellenlänge. Durch Vergleich der mit der Meßwellenlänge des Wassers gemessenen Strahlungsintensität mit der Strahlungsintensität der Vergleichswellenlänge wird ein Extinktionssignal gewonnen, das ein Maß für den absoluten Gehalt des Wassers in der Papierbahn darstellt. Dieses Meßsignal für den absoluten Gehalt des Wassers ist jedoch durch eine von der Opazität (innere Streuung) des Papiers hervorgerufene Verlängerung des Infrarotstrahlungswegs verfälscht. Zur Ermittlung des wahren absoluten Gehalts des Wassers in der Papierbahn wird daher das für Wasser gewonnene Meßergebnis mit einer die strukturellen Eigenschaften der Mischung aus Wasser und Cellulose berücksichtigenden Korrekturgröße multipliziert. Diese Korrekturgröße wird dadurch gebildet, daß das mittels Beta-Strahlung für das gesamte Flächengewicht der Materialbahn erhaltene Meßergebnis in Vergleich gesetzt wird mit dem mittels Infrarotstrahlung erhaltenen Meßsignal für das Flächegewicht von Cellulose. Diese Bildung der Korrekturgröße erfolgt unter der Annahme, daß das durch Infrarotmessung gewonnene Meßsignal für das Flächengewicht des Wassers im gleichen Verhältnis verfälscht ist wie das durch Infrarotmessung gewonnene Meßsignal für die Cellulose und daß das mittels Beta-Strahlung gewonnene Meßergebnis für das gesamte Flächengewicht der Materialbahn wegen des prozentual geringen Anteils des Wassers am gesamten Flächengewicht mit dem wahren Flächengewicht der Cellulose übereinstimmt. Von Bedeutung in diesem Zusammenhang ist noch, daß die Messung mittels der Beta-Strahlung durch die strukturellen Eigenschaften der Materialbahn bekanntlich nicht beeinflußt wird.

Bei dem bekannten Stand der Technik nach der DE-PS 29 10 673 werden bei der Infrarotstrahlungsmessung zum Eliminieren wellenlängenabhängiger Streuverluste von Infrarotstrahlung vorzugsweise zwei Vergleichswellenlängen angewendet. Mit Hilfe der beiden Vergleichswellenlängen ist es möglich, einen korrigierten Bezugswert für die mit den Meßwellenlängen erhaltenen Meßsignale zu ermitteln.

Das Vierstrahl-Meßverfahren nach der DE-PS 29 10 673 soll nicht nur für die Messung des Gehaltes von Wasser in Papier einsetzbar sein, sondern allgemein für Messungen an dünnen Materialbahnen, bei denen der Gehalt eines Stoffes im Material gemessen werden soll, unter de Voraussetzung, daß der zu messende Stoff mit den übrigen Stoffen des Materials gemischt vorliegt und derjenige weitere Stoff, von dem eine Meßwertverfälschung ausgehen kann, ebenfalls wie der zu messende Stoff eine ausgeprägte Absorptionsbande für Infrarotstrahlung hat. Das bekannte Meßverfahren ließe sich daher auch zur Messung des Gehalts einer Füllstoffkomponente in einer Materialbahn, insbesondere Papierbahn, heranziehen, sofern die Füllstoffkomponenten eine ausgeprägte Absorptionsbande im Infrarotstrahlungsbereich zeigt. Es tritt allerdings das Problem auf, daß bei einer in einer Papierbahn vorhandenen Füllstoffkomponente nicht mehr die Näherung gilt, daß das mittels der Beta-Strahlung gemessene gesamte Flächengewicht der Papierbahn mit dem wahren Flächengewicht der Cellulose übereinstimmt. Dies gilt um so mehr, wenn zusätzlich zu einer ersten Füllstoffkomponente noch eine zweite Füllstoffkomponente in dem Materialgemisch vorliegt. Darüber hinaus hat im allgemeinen die in einer Papierbahn vorgesehen zweite Füllstoffkomponente keine ausgeprägte Absorptionsbande im Infrarotstrahlungsbereich. Das aus der DE-PS 29 10 673 bekannte Meßverfahren ist daher zur selektiven Füllstoffmessung an Materialbahnen mit zwei Füllstoffkomponenten nicht geeignet.

Es besteht in der Papierindustrie ein allgemeines großes Bedürfnis an einer Meßeinrichtung, die die Aufteilung von zwei Füllstoffkomponenten, beispielsweise von Kaolin und Calciumcarbonat, an laufenden Bahnen aus gestrichenem Papier bestimmen kann. Dieses Bedürfnis liegt insbesondere dann vor, wenn dem zur Herstellung der Papierbahn benutzten Papierbrei in ungeregelter Weise als Papierabfall und Ausschußpapier zugeführt werden oder pigmentiertes gestrichenes Papier als Ausschub in den Rohpapierbrei zurückgeführt wird und dabei die genaue Kenntnis über die Füllstoffzusammensetzung verlorengeht.

Andererseits enthalten die zur Papierherstellung benutzen Füllstoffe in der Regel Verbindungen aus Metallen wie Aluminium in Kaolin und Satinweiß, Calcium in Calciumcarbonat und Satinweiß, Titan in Titandioxid und Barium in Bariumsulfat. Solche Metalle haben bekanntlich in einem Strahlungsenergiebereich von einigen keV charakteristische Absorptionseigenschaften, die sich zur hochselektiven Messung der betreffenden einzelnen Füllstoffe, im wesentlichen unabhängig von der Füllstoffzusammensetzung, anbieten, und zwar in Gegenwart des Trägerstoffes Cellulose und auch in der Papierbahn enthaltener Feuchtigkeit.

Diese Eigenschaften der Füllstoffe bilden die Grundlage für das bereits oben erwähnte, die Röntgenfluoreszenzanalyse ausnutzende Meßverfahren.

Das Problem der selektiven Füllstoffmessung ließe sich prinzipiell auch dadurch lösen, daß beispielsweise für zwei verschiedene, in der Materialmischung vorhandene Füllstoffe zwei voneinander unabhängige Messungen durchgeführt und dann ein System aus zwei linearen Gleichungen mit zwei unbekannten Größen gelöst wird, bei denen es sich jeweils um den Gehalt der beiden Füllstoffe handelt. Die Möglichkeit der Abstimmung der Energie einer Bremsstrahlung erzeugenden Röntgenstrahlung scheint eine Lösung für das aufgezeigte Problem zu bieten. Es könnten zwei Füllstoffmeßeinrichtungen mit in geeigneter Weise abgestimmten Energiespektren eingesetzt weden. Die eine Meßeinrichtung könnte auf den Energiebereich bei der Resonanzstelle der einen Füllstoffkomponente und die andere auf den Energiebereich bei der Resonanzstelle der anderen Füllstoffkomponente abgestimmt sein. Im Bereich der Resonanzen unterscheiden sich nämlich die jeweiligen Absorptionskoeffizienten am stärksten voneinander, so daß man bezüglich der Selektivität optimale Verhältnisse erhielte.

Das Emissionsspektrum einer Bremsstrahlung emittierenden Röntgenstrahlungsquelle ist allerdings äußerst breit. Die Halbwertbreite des Spektrums beträgt etwa 1/3 der Maximalenergie. Dieses breite Spektrum der Bremsstrahlung hat zur Folge, daß sich der Absorptionskoeffizient des zu messenden Stoffes mit zunehmendem Gewicht ändert, weil ein höherer Anteil der Strahlung niedriger Energie absorbiert wird. Dies bedeutet, daß das Absorptionsgesetz als Funktion höherer Ordnung empirisch bestimmt werden müßte. Das Absorptionsgesetz einer die Röntgenbremsstrahlung anwendenden Füllstoffmeßeinrichtung wäre daher system-abhängig. Der Vorteil der Abstimmbarkeit der Strahlungsenergie bei einer mit Bremsstrahlung arbeitenden Füllstoffmeßeinrichtung würde somit dadurch vollkommen aufgehoben werden, daß das zu lösende Gleichungssystem nichtlineare Terme enthielte. Darüber hinaus stellt sich das Problem der Instabilität der Messung infolge von Drift in der Hochspannungsversorgung von Röntgenröhren.

Der Erfindung liegt die Aufgabe zugrunde, ein Verfahren zur Messung des Flächengewichts von Stoffen in einer laufenden Papierbahn anzugeben, mit Hilfe dessen unter Einsatz einer möglichst einfachen und praktikablen Technik der Gehalt der einzelnen Füllstoffkomponenten in einer die Papierbahn ausmachenden Materialmischung mit hoher Genauigkeit ermittelt werden kann. Zur Lösung dieser Aufgabe wird die im Anspruch 1 angegebene Erfindung vorgeschlagen.

3

EP 0 272 645 B1

Das erfindungsgemäße Verfahren ermöglicht die selektive Ermittlung des Flächengewichts bzw. Gehalts von zwei Füllstoffkomponenten in einer aus einer Materialmischung bestehenden, laufenden Papierbahn, die im folgenden auch einfach Materialbahn genannt wird, unter der Voraussetzung, daß wenigstens die eine der beiden Füllstoffkomponenten eine ausgeprägte Absorptionsbande in dem Infrarotstrahlungsbereich hat. Dies ist für die erste Füllstoffkomponente aus Kaolin und/ oder Talkum erfüllt. Dadurch ist es möglich, mittels der Infrarotstrahlung ein von den übrigen Stoffen der Materialbahn im wesentlichen unabhängiges Meßsignal für den absoluten Gehalt oder das Flächengewicht der ersten Füllstoffkomponente zu erhalten, das allerdings wegen der in der Materialbahn auftretenden inneren Streuung verfälscht ist. Zur Bildung eines die Meßwertverfälschung berücksichtigenden Korrekturfaktors stützt sich die Erfindung auf die gleichzeitige Anwendung von Infrarotstrahlung, Beta-Strahlung und einer charakteristischen Röntgenstrahlung an der zu messenden Materialbahn. Die Verarbeitung der mit diesen Strahlung gewonnenen Meßsignale beruht auf der Erkenntnis, daß ein die tatsächlichen Umstände der inneren Streuung oder Opazität der Materialbahn Rechnung tragender Korrekturfaktor dadurch gewonnen werden kann, daß zwischen dem mittels der Beta-Strahlung gewonnenen, das gesamte Flächengewicht der Materialbahn darstellenden Meßsignal und einem Summensignal das Verhältnis gebildet wird, wobei das Summensignal durch Aufsummieren der mittels der IR-Strahlung gewonnenen Meßsignale für das Flächengewicht aller in der Materialbahn vorkommender Stoffe gewonnen wird. Die hierbei auftretende Schwierigkeit, daß für die zweite Füllstoffkomponente mangels einer ausgeprägten Absorptionsbande im Infrarotstrahlungsbereich ein solches Meßsignal nicht erhalten werden kann, wird durch den weiteren Ansatz überwunden, daß ein das nicht meßbare Signal ersetzendes fiktives oder theoretisches Signal für das Flächengewicht der zweiten Füllstoffkomponente durch die Opazität der Materialbahn im gleichen Verhältnis verfälscht sein soll wie das mittels Infrarotstrahlung erfaßbare Meßsignal für das Flächengewicht der ersten Füllstoffkomponente.

Die oben erläuterten Annahmen gestatten es dann, unter Heranziehung der mittels der drei Strahlungsarten erhaltenen Meßsignale den Korrekturfaktor zu bilden, der nicht nur auf das mittels der Infrarotstrahlung gewonnene Meßsignal für das Flächengewicht der ersten Füllstoffkomponente, sondern auf jedes der mittels Infrarotstrahlung gewonnenen Meßsignale für das Flächengewicht eines in der Materialbahn enthaltenen und eine ausgeprägte Absorptionsbande im Infrarotstrahlungsbereich aufweisenden Stoffes angewendet werden kann.

Die Anwendung der Infrarotstrahlung und die Gewinnung der mit Hilfe der verschiedenen Meßwellenlängen und vorzugsweise zwei Vergleichswellenlänge gewonnenen Meßsignale für die Flächengewichte der mittels Infrarotstrahlung meßbaren Stoffe der Materialbahn kann im wesentlichen so erfolgen, wie es in der DE-PS 29 10 673 beschrieben ist. Bei Anwendung von zwei Vergleichswellenlängen kann ein genauerer Bezugswert für die bei den Meßwellenlängen gemessenen Strahlungsintensitätssignale gebildet werden. Sollte es erforderlich sein, wird zusätzlich die für einen Stoff mit der von diesem Stoff absorbierten Meßwellenlänge der Infrarotstrahlung gemessene Strahlungsintensität bezüglich einer Intensitätsverfälschung korrigiert, die bei der Meßwellenlänge dieses Stoffes durch einen einer anderen Meßwellenlänge zugeordneten Stoff verursacht wird.

Die Messung des gesamten Flächengewichtes einer Materialbahn mit der Beta-Strahlung ist eine an sich bekannte und dem Fachmann wohlvertraute Messung. Als Korpuskularstrahlung wird die Beta-Strahlung im Gegensatz zur Infrarotstrahlung durch die strukturellen Eigenschaften der Materialmischung der zu messenden Materialbahn nicht beeinflußt. Die Beta-Strahlung liefert daher im allgemeinen ein Meßsignal für das gesamte Flächengewicht der Materialbahn. Bei Messung von einer Papierbahn bedeutet dies, daß mittels der Beta-Strahlung der Celluloseanteil, die beiden Füllstoffkomponentenanteile und der Wasseranteil des Papiers erfaßt werden.

Bei der charakteristischen Röntgenstrahlung handelt es sich vorzugsweise um die bei 5,9 keV auftretende Quantenstrahlung der natürlichen radioaktiven Strahlungsquelle Fe-55. Die Fe-55-Strahlungsquelle ist an sich dem Fachmann wohlbekannt, und es stehen ihm auch entsprechende Strahlungsdetektoren zur Verfügung.

Im Gegensatz zu der einen weiten Spektralbereich überdeckenden Röntgenbremsstrahlung besteht die charakteristische Röntgenstrahlung aus einzelnen charakteristischen Linien. Obgleich die charakteristische Röntgenstrahlung bzw. die von der Fe-55-Strahlungsquelle emittierte Quantenstrahlung nicht auf eine spezifische Energie abgestimmt werden kann und daher zur Lösung der erfindungsgemäßen Aufgabe weniger als die abstimmbare Röntgenbremsstrahlung geeignet zu sein scheint, sind es gerade die einzelnen charakteristischen Linien der charakteristischen Röntgenstrahlung bzw. die monenergetische Eigenschaft der stabilen Fe-55-Strahlungsquelle in Kombination mit der Infrarotstrahlungsmessung und der Beta-Strahlungsmessung für den erzielten Gesamterfolg der Erfindung von wesentlicher Bedeutung. Für die einzelnen charakteristischen Linien der charakteristischen Röntgenstrahlung bzw. die monenergetische Quantenstrahlung der Fe-55-Strahlungsquelle ist das Absorptionsgesetz streng exponentiell mit wohl defi-

4

nierten Absorptionskoeffizienten, die tabelliert sind und ohne weiteres auch empirisch bestimmt werden können. Aus dem streng exponentiellen Absorptionsgesetz folgt, daß sich alle Strahlungsextinktionsbeiträge der verschiedenem Stoffe der untersuchten Materialbahn mathematisch zu einem resultierenden Extinktionssignal aufaddieren. Im Falle der einzelnen charakteristischen Linien der charakteristischen Röntgenstrahlung bzw. der monochromatischen Energie der Fe-55-Strahlungsquelle kann die von jedem Stoff der Materialbahn verursachte Extinktion von der gemessenen Gesamtextinktion subtrahiert werden. Obgleich somit mit der charakteristischen Röntgenstrahlung allein keine selektive Messung durchgeführt werden kann, ist bei der erfindungsgemäßen selektiven Füllstoffmessung die Heranziehung des mit der charakteristischen Röntgenstrahlung gewonnenen Meßsignals in Verbindung mit den mittels der Beta-Strahlung und Infrarotstrahlung gewonnenen Meßsignalen zur Korrektur der mittels der Infrarotstrahlung gewonnenen Meßsignale zum Erreichen des erfindungsgemäßen Zwecks von entscheidender Bedeutung.

Für den Fall der berührungslosen selektiven Füllstoffmessung an laufenden Papierbahnen nutzt die Erfindung die Erkenntnis aus, daß sowohl der Füllstoff Kaolin als auch einer der hauptsächlichsten Ersatzfüllstoffe für Kaolin, nämlich Talkum, ausgeprägte, aber voneinander getrennte Absorptionsbanden im Infrarotstrahlungsbereich haben. Dadurch ist es möglich, für die erste Füllstoffkomponente ein zwar korrekturbedürftiges, jedoch von der zweiten Füllstoffkomponente unabhängiges Meßsignal zu gewinnen. Handelt es sich bei der zweiten Füllstoffkomponente beispielsweise um den Füllstoff Calciumcarbonat, der mittels Infrarotstrahlung nicht erfaßt werden kann, ist es mit Hilfe des erfindungsgemäßen Meßverfahrens dennoch möglich, die Aufteilung zwischen diesen beiden Füllstoffkomponenten genau zu bestimmten. Das durch Anwendung der Röntgenstrahlmessung erhaltene Meßsignal stellt zwar die durch Kaolin und/oder Talkum und Calciumcarbonat sowie Cellulose und Wasser hervorgerufene Gesamtextinktion dar, jedoch läßt sich infolge der monochromatischen und stabilen Energie der benutzten Röntgenstrahlung der für diese Strahlungsenergie auftretende Extinktionsbeitrag der in der Papierbahn vorhandenen genau berechnen. Die für die Energie der Röntgenstrahlung auftretenden Absorptionskoeffizienten von Kaolin und/oder Talkum, Calciumcarbonat, Cellulose und Wasser sind wohldefiniert und bekannt. Im Hinblick auf die exponentielle Absorption der die Papierbahn durchsetzenden Röntgenstrahlung ist die Differenz zwischen der mit der charakteristischen Röntgenstrahlung gemessenen Gesamtextinktion und den für Cellulose, Kaolin und/oder Talkum und Wasser berechneten und aufsummierten Extinktionsbeiträgen ausschließlich auf die Absorptionswirkung der zweiten Füllstoffkomponente Calciumcarbonat zurückzuführen. Mann kann daher ohne weiteres die Flächengewichte für die beiden Füllstoffkomponenten erhalten. Damit ist es auch möglich, die Aufteilung zwischen den beiden Füllstoffkomponenten Kaolin und Calciumcarbonat sowie den gesamten Füllstoffgehalt anzugeben.

Die obigen Ausführungen gelten selbstverständlich auch für den Fall, daß der Füllstoff Calciumcarbonat durch Titandioxid, Satinweiß oder Bariumsulfat ersetzt ist. Für den Fall, daß beide Füllstoffe Kaolin und Talkum gleichzeitig vorhanden sind, erfolgt die Messung der ersten Füllstoffkomponente mittels Infrarotstrahlung sowohl mit einer von Kaolin absorbierten Meßwellenlänge als auch mit einer von Talkum absorbierten Meßwellenlänge.

Das erfindungsgemäße Meßverfahren befriedigt ein bei der Papierherstellung auftretendes dringendes Bedürfnis, das den Papierhersteller in die Lage versetzt, bei laufender Kenntnis der Aufteilung von zwei verschiedenen Füllstoffkomponenten die Füllstoffzusammensetzung des hergestellten Papiers so zu steuern, daß unter Verwendung der jeweils wirtschaftlichsten Füllstoffe das gesteckte Qualitätsziel erreicht wird. Die aufgezeigte Lösung ist praktisch leicht zu realisieren und zeichnet sich noch durch den besonderen Vorteil aus, daß eine Eichung der Röntgenstrahlungsquelle in Abhängigkeit von den zu messenden Papiersorten nicht erforderlich ist.

Zur weiteren Erlauterung der Erfindung wird auch auf die beigefügten Zeichnungen Bezug genommen. Es zeigt:

FIG. 1 ein Diagramm, das für zwei Füllstoffe die Abhängigkeit des Absorptionskoeffizienten von der Strahlungsenergie im Bereich von einigen keV veranschaulicht,

FIG. 2 ein Diagramm zur Veranschaulichung der relativen Intensität einer Rönten-Bremsstrahlung und der monoenergetischen Röntgenstrahlung einer Fe-55-Strahlungsquelle in Abhängigkeit von der Strahlungsenergie,

FIG. 3 Teile von Nahinfrarot-Spektren zur Veranschaulichung der Absorption verschiedener Substanzen von Papier in Abhängigkeit von der Wellenlänge,

FIG. 4 eine Schemadarstellung einer möglichen Meßanordnung zur Durchführung des erfindungsgemäßen Verfahrens und

FIG. 5 ein vereinfachtes Signalflußbild zur Erläuterung des erfindungsgemäßen Meßverfahrens.

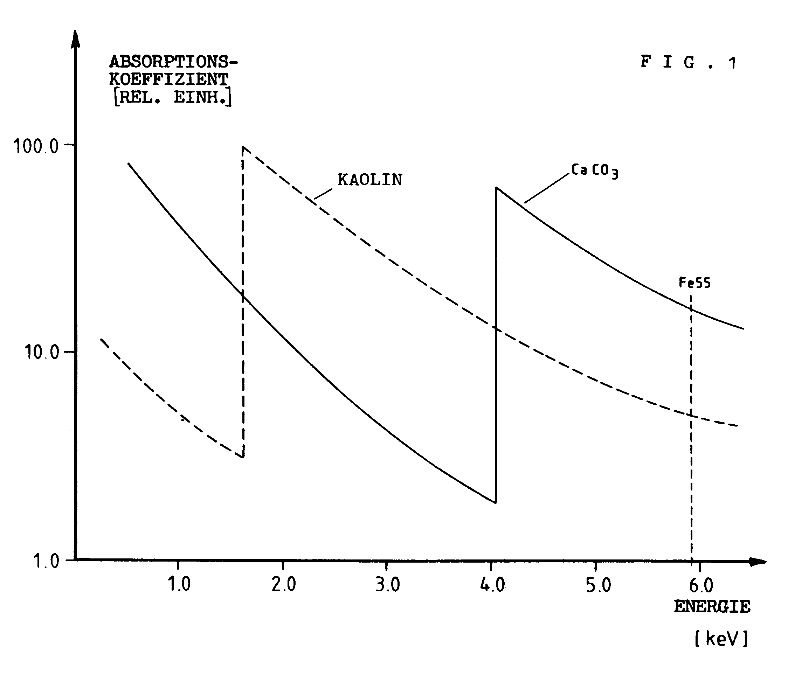

FIG. 1 zeigt den typischen Verlauf des Absorptionskoeffizienten von in Papierbahnen vorkommenden Füllstoffen wie Kaolin und Calciumcarbonat, die Verbindungen von Metallen enthalten. Die dargestellten

EP 0 272 645 B1

Absorptionskanten sind für die Metallatome der betreffenden Füllstoffe charakteristisch. In das Diagramm von FIG. 1 ist noch im Vergleich zur Lage der spezifischen Absorptionskanten von Kaolin und Calciumcarbonat die Lage der Quantenstrahlung der natürlichen radioaktiven Strahlungsquelle Fe-55 eingezeichnet, die vorzugsweise für die erfindungsgemäßen Zwecke verwendet wird.

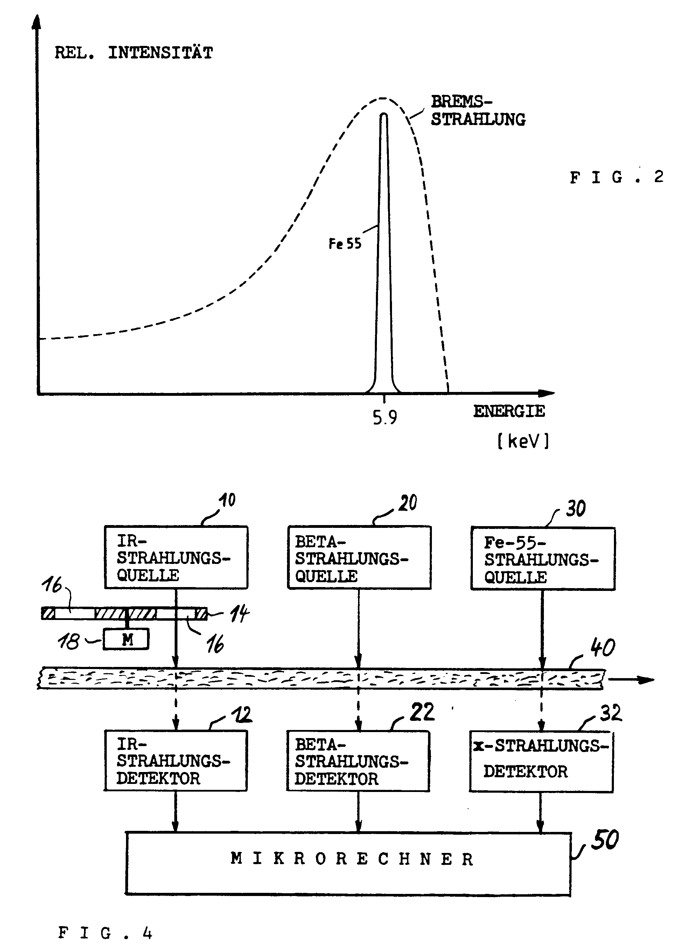

FIG. 2 veranschaulicht das bei 5,9 KeV liegende, monochromatische Energiespektrum der Fe-55-Strahlungsquelle im Vergleich zu einer Röntgenbremsstrahlung, die mit Hilfe der Beschleunigungsspannung im Maximum auf die monochromatische Strahlungsenergie der Fe-55-Strahlungsquelle abgestimmt worden ist.

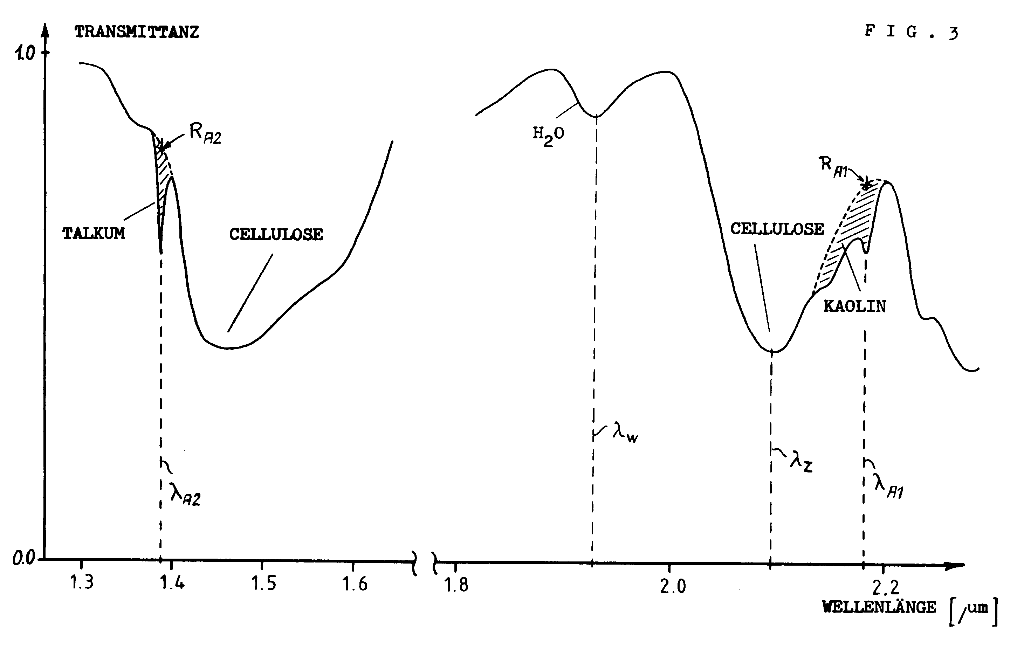

FIG. 3 zeigt die Absorption verschiedener Substanzen oder Stoffe von Papier im Nahinfrarotbereich des elektromagnetischen Spektrums. Außer dem Trägerstoff Cellulose enthält die Papierbahn die Füllstoffe Kaolin und Talkum sowie Feuchtigkeit bzw. Wasser. Wie man erkennen kann, weisen im Infrarotstrahlungsbereich nicht nur Cellulose und Wasser, sondern auch Kaolin und Talkum ausgeprägte Absorptionsspektren auf, die durch schraffierte Flächen kenntlich gemacht sind.

Sollen an dieser Papierbahn Extinktionsmessungen vorgenommen werden, ordnet man in an sich bekannter Weise den Stoffen der Papierbahn, deren Extinktion gemessen werden soll, jeweils eine Meßwellenlänge zu, die von dem betreffenden Stoff absorbiert wird. In FIG. 3 sind demzufolge eine mögliche Meßwellenlänge $\lambda_Z$ für Cellulose, $\lambda_W$ für Wasser, $\lambda_{A1}$ für Kaolin und $\lambda_{A2}$ für Talkum eingezeichnet. Für Kaolin und Talkum sind auch die jeweils zugeordneten Nullpunkte $R_{A1}$ und $R_{A2}$ eingezeichnet. Auf die Einzeichnung der Nullpunkte für Cellulose und Wasser sowie auf die Einzeichnung von zwei Vergleichswellenlängen, die beispielsweise bei 1,3 und 1,8 $\mu$m liegen könnten, wurde der besseren Übersicht halber verzichtet. Aus dem Stand der Technik nach der DE-PS 29 10 673 ist es bekannt, wie aus den Intensitätsmessungen mit den beiden Vergleichswellenlängen die Nullpunkte für die Meßwellenlängen gewonnen werden.

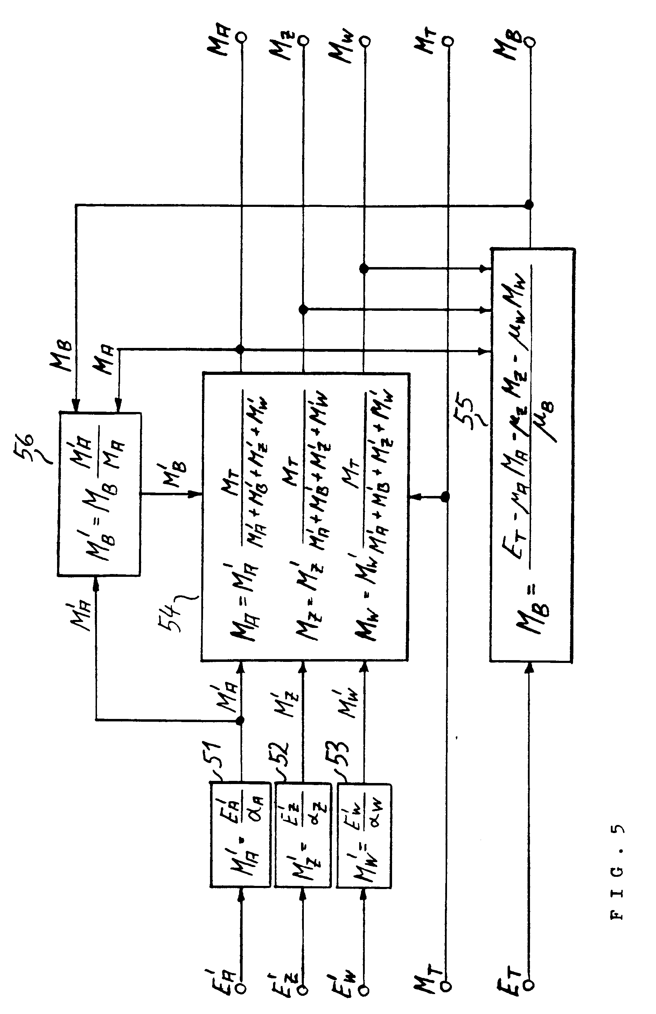

Die in FIG. 4 dargestellte Meßanordnung enthält eine Infrarot-Strahlungseinrichtung mit einer Infrarot-Strahlungsquelle 10, einem von einem Motor 18 angetriebenen Filterrad 14 und einem Infrarot-Strahlungsdetektor 12. Eine Beta-Strahlungsmeßeinrichtung enthält eine Beta-Strahlungsquelle 20 und einen Beta-Strahlungsdetektor 22. Eine monoenergetische Röntgen-oder z-Strahlungsmeßeinrichtung enthält eine Fe-55-Strahlungsquelle 30 und einen x-Strahlungsdetektor 32.

Eine dünne zu messende Papierbahn 40, die sich kontinuierlich in der Richtung eines eingezeichneten Pfeils bewegt, wird von den Meßstrahlen der einzelnen Meßeinrichtungen durchsetzt. Das Filterrad 14 weist bei einem betrachteten Ausführungsbeispiel fünf Filter 16 auf, von denen drei für jeweils eine Meßwellenlänge und zwei für jeweils eine Vergleichswellenlänge gedacht sind. Die von den Strahlungsdetektoren 12, 22 und 32 erhaltenen elektrischen Meßsignale werden einem Mikrorechner 50 zugeführt, der die Meßsignale bzw. daraus abgeleitete proportionale Signale grundsätzlich in einer Weise verarbeitet, wie es aus dem Signalflußbild nach FIG. 5 hervorgeht.

Für die nachfolgende Betrachtung wird angenommen, daß die zu messende Papierbahn 40 neben dem mit Z bezeichneten Trägerstoff Cellulose und dem mit W bezeichneten weiteren Stoff Wasser eine mit A bezeichnete erste Füllstoffkomponente und eine mit B bezeichnete zweite Füllstoffkomponente enthält. Bei der ersten Füllstoffkomponente A handelt es sich bei dem betrachteten Ausführungsbeispiel um Kaolin und bei der zweiten Füllstoffkomponente um Calciumcarbonat. Demzufolge wählt ein erstes Filter der fünf Filter 16 eine auf das Absorptionsspektrum von Kaolin abgestellte Meßwellenlänge $\lambda_A$ ($\lambda_{A1}$ nach FIG. 3) aus, ein zweites Filter eine auf der Absorptionsspektrum von Cellulose abgestellte Meßwellenlänge $\lambda_Z$ und ein drittes Filter eine auf das Absorptionsspektrum von Wasser abgestellte Meßwellenlänge $\lambda_W$. Die beiden noch verbleibenden Filter wählen die Vergleichswellenlängen $\lambda_1$ und $\lambda_2$ aus. Nach der bekannten Beziehung

$$E' = -\ell n \frac{I}{I_0} \qquad\qquad (1)$$

werden aus den vom Infrarot-Strahlungsdetektor 12 erfaßten Strahlungsintensitätssignalen ein Extinktionssignal $E'_A$ für Kaolin, ein Extinktionssignal $E'_Z$ für Cellulose und ein Extinktionssignal $E'_W$ für Wasser erhalten. In der vorstehenden Gleichung (1) bedeuten I die vom Detektor 12 bei einer der Meßwellenlängen gemessene Strahlungsintensität, $I_0$ eine der betreffenden Meßwellenlänge zugeordnete Nullintensität, die aus den vom Detektor 12 bei den Vergleichswellenlängen $\lambda_1$ und $\lambda_2$ gemessenen Strahlungsintensitäten ermittelt wird, und E' die bei der betreffenden Meßwellenlänge für den zugeordneten Stoff gemessene Extinktion.

Nach der bekannten Beziehung

6

$$M' = \frac{E'}{a} \qquad (2)$$

erhält man aus den gemessenen Extinktionssignalen für die einzelnen Stoffe unter Verwendung des jeweils zugeordneten Absorptionskoeffizienten jeweils ein Meßsignal $M'_A$ für das Flächengewicht von Kaolin, $M'_Z$ für das Flächengewicht von Cellulose und $M'_W$ für das Flächengewicht von Wasser. In der Gleichung (2) ist a der Absorptionskoeffizient des betreffenden Stoffes bei der dem Stoff zugeordneten Meßwellenlänge der Infrarotstrahlung. Dementsprechend ist $a_A$ der Absorptionskoeffizient von Kaolin bei der Meßwellenlänge $\lambda_A$, $a_Z$ der Absorptionskoeffizient von Cellulose bei der Meßwellenlänge $\lambda_Z$ und $a_W$ der Absorptionskoeffizient für Wasser bei der Meßwellenlänge $\lambda_W$. Diese Absorptionskoeffizienten sind an sich bekannt und können auch experimentell leicht bestimmt werden.

Für die zweite Füllstoffkomponente Calciumcarbonat kann man mangels einer Absorptionsbande im Infrarotstrahlungsbereich ein Meßsignal für das Flächengewicht von Calciumcarbonat nicht erhalten.

Wie es im einzelnen in der DE-PS 29 10 673 dargelegt ist, entsprechen die mit Infrarotstrahlungsmessung gewonnenen Meßsignale M' nicht dem tatsächlichen Flächengewicht M der jeweils in der Papierbahn gemessenen Stoffe. Es ist vielmehr erforderlich, das jeweilige Meßsignal M' mit einem Korrekturfaktor K zu multiplizieren, um das jeweilige tatsächliche Flächengewicht M zu erhalten.

Es hat sich experimentell bestätigt, daß für den Korrekturfaktor K der folgende Ansatz gemacht werden kann:

$$K = \frac{M_T}{M'_A + M'_B + M'_Z + M'_W} \qquad (3)$$

In der Gleichung (3) ist $M_T$ das mittels der Beta-Strahlung gewonnene Meßsignal für das gesamte Flächengewicht der Papierbahn. Das Meßsignal $M_T$ wird in an sich bekannter Weise aus den vom Beta-Strahlungsdetektor 22 gelieferten Strahlungsintensitätssignalen gewonnen, wobei die Referenzintensität bei nicht vorhandener Papierbahn bemessen wird. Das Signal $M'_B$ soll den von der zweiten Füllstoffkomponente Calciumcarbonat hervorgerufenen Anteil an der Meßwertverfälschung der mittels Infrarotstrahlung gemessenen Meßsignale M' berücksichtigen und ist zunächst unbekannt.

Zur Bestimmung des Signals $M'_B$ wird angenommen, daß in dem fiktiven oder theoretischen Meßsignal $M'_B$ für das Flächengewicht der nicht mittels Infrarotstrahlung meßbaren zweiten Füllstoffkomponente Calciumcarbonat eine Verfälschung enthalten ist, die verhältnisgleich zu der Verfälschung in dem Meßsignal $M'_A$ für das Flächengewicht der mittels Infrarotstrahlung meßbaren ersten Füllstoffkomponente Kaolin ist. Hieraus folgt die Beziehung

$$\frac{M'_B}{M_B} = \frac{M'_A}{M_A} \qquad (4)$$

Darin bedeuten $M_A$ das wahre Flächengewicht der ersten Füllstoffkomponente Kaolin und $M_B$ das wahre Flächengewicht der zweiten Füllstoffkomponente Calciumcarbonat.

Nach der bekannten Beziehung

$$E_T = - \ell n \, \frac{U}{U_o} \qquad (5)$$

gewinnt man aus den vom x-Strahlungsdetektor 32 bereitgestellten elektrischen Strahlungsintensitätssignalen ein Meßsignal $E_T$ für die Gesamtextinktion der Papierbahn bei der charakteristischen Röntgenstrahlung der Fe-55-Quelle. In der Gleichung (5) bedeuten U die Stärke des vom Detektor 32 abgegebenen elektrischen Meßsignals bei im Meßspalt befindlicher Papierbahn und $U_o$ die Stärke des vom Detektor 32 abgegebenen elektrischen Meßsignals Jedoch ohne Papierbahn.

Unter Beachtung des für die benutzte x-Strahlungsquelle geltenden exponentiellen Absorptionsgesetzes kann man für das von dieser Meßeinrichtung gelieferte Meßsignal $E_T$ die folgende Gleichung aufstellen:

$$E_T = \mu_A M_A + \mu_B M_B + \mu_Z M_Z + \mu_W M_W \qquad (6)$$

Hierin bedeuten $M_A$ das Flächengewicht des in der Papierbahn vorliegenden Füllstoffes Kaolin, $M_B$ das Flächengewicht des in der Papierbahn vorliegenden Füllstoffes Calciumcarbonat, $M_Z$ das Flächengewicht des in der Papierbahn vorliegenden Trägerstoffes Cellulose, $M_W$ das Flächengewicht des in der Papierbahn enthaltenen Wassers, $\mu_A$ den Absorptionskoeffizienten von Kaolin bei der x-Meßstrahlungsenergie von 5,9 keV, $\mu_B$ den Absorptionskoeffizienten von Calciumcarbonat bei der x-Meßstrahlungsenergie von 5,9 keV, $\mu_Z$ den Absorptionskoeffizienten von Cellulose bei der x-Meßstrahlungsenergie von 5,9 keV und $\mu_W$ den Absorptionskoeffizienten von Wasser bei der x-Meßstrahlungsenergie von 5,9 keV. Die genannten Absorptionskoeffizienten $\mu$ sind bekannt und können auch ohne weiteres experimentell ermittelt werden.

Die Gleichung (6) läßt sich in die nachfolgende Gleichung (7) umformen:

$$M_B = \frac{E_T - \mu_A M_A - \mu_Z M_Z - \mu_W M_W}{\mu_B} \qquad (7)$$

Unter Bezugnahme auf das Signalflußdiagramm nach FIG. 5 kann man somit mit den dort angegebenen Kalkulationsblöcken 51 bis 56 unter Verwendung der von den Ausgangssignalen der Detektoren 12, 22 und 32 gewonnenen Meßsignale $M'_A$, $M'_Z$, $M'_W$, $M_T$ und $E_T$ den Korrekturfaktor K zur Korrektur der mittels Infrarotstrahlung gewonnenen Meßsignale $M'_A$, $M'_Z$ und $M'_W$ ermitteln. Wie man aus FIG. 5 erkennen kann, handelt es sich bei dem zunächst unbekannten Signal $M'_B$ um ein zum Kalkulationsblock 54 zurückgeführtes Signal, zu dessen Bildung das Ausgangssignal $M_A$ des Kalkulationsblocks 54 selbst und das Ausgangssignal $M_B$ des Kalkulationsblocks 55 herangezogen werden, wobei dem Kalkulationsblocks 55 seinerseits neben dem Meßsignal $E_T$ die Ausgangssignale $M_A$, $M_W$ und $M_Z$ des Kalkulationsblocks 54 zugeführt werden.

Die ermittelten Flächengewichte $M_A$ und $M_B$ für Kaolin und Calciumcarbonat können dann in üblicher Weise mit dem gesamten Flächengewicht $M_T$ der Papierbahn verknüpft werden, um beispielsweise den prozentualen Gehalt der einzelnen Füllstoffkomponente oder den prozentualen Aschegehalt in der Papierbahn anzugeben. Dazu können beispielsweise die folgenden Beziehungen benutzt werden:

$$P_A = \frac{100 \cdot M_A}{M_T} \qquad (8)$$

$$P_B = \frac{100 \cdot M_B}{M_T} \qquad (9)$$

$$P_H = \frac{100 (M_A + M_B)}{M_T} \qquad (10)$$

In den Gleichungen (8) bis (10) bedeuten $P_A$ der prozentuale Gehalt der ersten Füllstoffkomponente in der Papierbahn, $P_B$ der prozentuale Gehalt der zweiten Füllstoffkomponente in der Papierbahn und $P_H$ der prozentuale Aschegehalt in der Papierbahn.

**Patentansprüche**

**1.** Verfahren zur Messung des Flächengewichts von Stoffen in einer laufenden Papierbahn aus einer Materialmischung mit Cellulose als Trägerstoff (Z), Kaolin und/oder Talkum als erste Füllstoffkomponente (A), Satinweiß, Calciumcarbonat, Titanoxid oder Bariumsulfat als zweite Füllstoffkomponente (B) und Wasser als weiterer Stoff (W),
**gekennzeichnet durch**

8

(a) Anwendung einer Infrarotstrahlung mit einer von dem Trägerstoff (Z) absorbierten Meßwellenlänge ($\lambda_Z$),einer von der ersten Füllstoffkomponente (A) absorbierten Meßwellenlänge ($\lambda_A$) und einer von dem weiteren Stoff (W) absorbierten Meßwellenlänge ($\lambda_W$) sowie wenigstens einer von diesen Stoffen (Z, A, B, W) im wesentlichen nicht absorbierten Vergleichswellenlänge ($\lambda_1$) und Messung der bei den einzelnen Meßwellenlängen ($\lambda_Z$, $\lambda_A$, $\lambda_W$) an der Materialbahn auftretenden Extinktionen ($E'_Z$, $E'_A$, $E'_W$) zur Gewinnung eines Meßsignals ($M'_Z$) für das Flächengewicht des Trägerstoffes (Z), eines Meßsignals ($M'_A$) für das Flächengewicht der ersten Füllstoffkomponente (A) und eines Meßsignals ($M'_W$) für das Flächengewicht des weiteren Stoffes (W),

(b) Anwendung einer Beta-Strahlung zur Gewinnung eines das gesamte Flächengewicht der Materialbahn (T) darstellenden Meßsignals ($M_T$),

(c) Anwendung einer von dem Trägerstoff (Z), den beiden Füllstoffkomponenten (A, B) und dem weiteren Stoff (W) absorbierten charakteristischen Röntgenstrahlung und Messung der bei der charakteristischen Röntgenstrahlung an der Materialbahn (T) auftretenden Gesamtextinktion zur Gewinnung eines die aufsummierten Extinktionsbeiträge der in der Materialbahn enthaltenen Stoffe darstellenden Meßsignals ($E_T$), und

(d) Bildung eines Korrekturfaktors (K) für das mittels der Infrarotstrahlung gewonnene Meßsignal ($M'_A$) für das Flächengewicht der ersten Füllstoffkomponente (A) durch Vergleich des mittels der Beta-Strahlung gewonnenen, das gesamte Flächengewicht der Materialbahn darstellenden Meßsignals ($M_T$) mit einem Summensignal aus den mittels Infrarotstrahlung gewonnenen Meßsignalen ($M'_Z$, $M'_A$, $M'_W$) für die Flächengewichte des Trägerstoffes (Z), der ersten Füllstoffkomponente (A) und des weiteren Stoffes (W) sowie aus einem theoretischen Meßsignal ($M'_B$) für das Flächengewicht der zweiten Füllstoffkomponente (B), wobei das theoretische Meßsignal ($M'_B$) unter zusätzlicher Heranziehung des mittels der charakteristischen Röntgenstrahlung gewonnenen, die Gesamtextinktion der Materialbahn darstellenden Meßsignals ($E_T$) mit der Annahme ermittelt wird, daß das theoretische Meßsignal ($M'_B$) für das Flächengewicht der zweiten Füllstoffkomponente (B) im gleichen Verhältnis verfälscht ist wie das mittels IR-Strahlung gewonnene Meßsignal ($M'_A$) für das Flächengewicht der ersten Füllstoffkomponente (A).

2. Verfahren nach Anspruch 1,
**dadurch gekennzeichnet,**
daß zum Ermitteln eines korrigierten Bezugswertes für die mit den Meßwellenlängen der Infrarotstrahlung gemessenen Strahlungsintensitäten zwei von den Stoffen der Materialbahn im wesentlichen nicht absorbierte Vergleichswellenlängen ($\lambda_1$, $\lambda_2$) angewendet werden.

3. Verfahren nach einem der Ansprüche 1 oder 2,
**dadurch gekennzeichnet,**
daß für den Fall, daß die erste Füllstoffkomponente aus den beiden Füllstoffen Kaolin und Talkum besteht, für jeden dieser beiden Füllstoffe eine von ihm absorbierte Meßwellenlänge ($\lambda_{A1}$, $\lambda_{A2}$) im Infrarotstrahlungsbereich angewendet wird.

4. Meßanordnung zur Durchführung des Verfahrens nach einem der vorstehenden Ansprüche,
**gekennzeichnet durch**
eine Kombination aus einer Mehrwellenlängen-Infrarotstrahlungsmeßeinrichtung (10 bis 18), einer Beta-Strahlungsmeßeinrichtung (20, 22), einer Röntgenstrahlungsmeßeinrichtung (30, 32) mit einer Fe-55-Strahlungsquelle sowie einem Mikrorechner (50) zur Auswertung der von den drei Meßeinrichtungen gewonnenen Meßsignale.

## Claims

1. Method of measuring the weight per unit area of substances in a moving paper web consisting of a stock mix with cellulose as base substance (Z), kaolin and/or talc as first filler component (A), satin white, calcium carbonate, titanium oxide or barium sulphate as second filler component (B) and water as a further substance (W), characterised by

(a) application of infrared radiation with a measurement wavelength ($\lambda_Z$) absorbed by the base substance (Z), a measurement wavelength ($\lambda_A$) absorbed by the first filler component (A) and a measurement wavelength ($\lambda_W$) absorbed by the further substance (W) and also at least one comparison wavelength ($\lambda_1$) substantially not absorbed by these substances (Z, A, B, W) and measurement of the extinctions ($E'_Z$, $E'_A$, $E'_W$) occurring at the web of material for the individual

9

measurement wavelengths ($\lambda_Z$, $\lambda_A$, $\lambda_W$) to obtain a measurement signal ($M'_Z$) for the weight per unit area of the base substance (Z), a measurement signal ($M'_A$) for the weight per unit area of the first filler component (A) and a measurement signal ($M'_W$) for the weight per unit area of the further substance (W),

(b) application of beta-radiation to obtain a measurement signal ($M_T$) representing the total weight per unit area of the web of material (T),

(c) application of a characteristic x-radiation absorbed by the base substance (Z), both filler components (A, B) and the further substance (W) and measurement of the total extinction occurring at the web of material (T) in the characteristic x-radiation to obtain a measurement signal ($E_T$) representing the sum of the extinction contributions of the substances contained in the web of material,

and

(d) formation of a correction factor (K) for the measurement signal ($M'_A$) obtained by means of the infrared radiation for the weight per unit area of the first filler component (A) by comparison of the measurement signal ($M_T$) representing the total weight per unit area of the web of material obtained by means of the beta-radiation with a sum signal derived from the measurement signals ($M'_Z$, $M'_A$, $M'_W$) obtained by means of infrared radiation for the weights per unit area of the base substance (Z), the first filler component (A) and the further substance (W) and also from a theoretical measurement signal ($M'_B$) for the weight per unit area of the second filler component (B), the theoretical measurement signal ($M'_B$) being determined with the additional use of the measurement signal ($E_T$) obtained by means of the characteristic x-radiation and representing the total extinction of the web of material, on the assumption that the theoretical measurement signal ($M'_B$) for the weight per unit area of the second filler component (B) is falsified in the same proportion as the measurement signal ($M'_A$) obtained by means of IR radiation for the weight per unit area of the first filler component (A).

2. Method according to claim 1, characterised in that two comparison wavelengths ($\lambda_1$, $\lambda_2$) substantially not absorbed by the substances in the web of material are employed for determining a corrected reference value for the radiation intensities measured with the measurement wavelengths of the infrared radiation.

3. Method according to either of claims 1 and 2, characterised in that in the event of the first filler component consisting of the two fillers kaolin and talc, there is employed for each of these two fillers a measurement wavelength ($\lambda_{A1}$, $\lambda_{A2}$) in the infrared radiation range which is absorbed by it.

4. Measuring system for carrying into effect the method according to any one of the preceding claims, characterised by a combination of multi-wavelength infrared radiation measuring equipment (10 to 18), beta-radiation measuring equipment (20, 22), x-radiation measuring equipment (30, 32) with an Fe55 radiation source and also a microcomputer (50) for evaluating the measurement signals obtained from the three measuring equipments.

**Revendications**

1. Procédé de mesure du grammage de matières dans une bande de papier défilante constituée d'un mélange de matériaux composé de cellulose comme matièresupport (Z), de kaolin et/ou de talc comme premier composant de charge (A), de blanc brillant, carbonate de calcium, oxyde de titane ou de sulfate de baryum comme deuxième composant de charge (B) et d'eau comme autre corps (W), caractérisé par

(a) l'utilisation d'un rayonnement infrarouge avec une longueur d'onde de mesure ($\lambda_Z$) absorbée par la matière-support (Z), une longueur d'onde de mesure ($\lambda_A$) absorbée par le premier composant de charge (A) et une longueur d'onde de mesure ($\lambda_W$) absorbée par l'autre corps (W) ainsi qu'au moins une longueur d'onde de comparaison ($\lambda_1$) qui n'est pour l'essentiel pas absorbée par ces matières (Z, A, B, W) et la mesure des extinctions ($E'_Z$, $E'_A$, $E'_W$) se produisant sur la bande de matériau aux différentes longueurs d'onde de mesure ($\lambda_Z$, $\lambda_A$, $\lambda_W$) afin d'obtenir un signal de mesure ($M'_Z$) représentant le grammage de la matière-support (Z), un signal de mesure ($M'_A$) correspondant au grammage du premier composant de charge (A) et un signal de mesure ($M'_W$) correspondant au grammage de l'autre corps (W),

(b) l'utilisation d'un rayonnement bêta pour obtenir un signal de mesure ($M_T$) représentant le grammage total de la bande de matériau (T),

(c) l'utilisation d'un rayonnement X caractéristique absorbé par la matière-support (Z), les deux composants de charge (A, B) et l'autre corps (W) et la mesure de l'extinction totale se produisant sur la bande de matériau (T) en présence du rayonnement X caractéristique afin d'obtenir un signal de mesure ($E_T$) représentant la somme des extinctions des matières contenues dans la bande de matériau, et

(d) la formation d'un facteur de correction (K) pour le signal de mesure ($M'_A$) obtenu au moyen du rayonnement infrarouge et représentant le grammage du premier composant de charge (A) en comparant le signal de mesure ($M_T$) obtenu au moyen du rayonnement bêta et représentant le grammage total de la bande de matériau avec un signal de somme résultant des signaux de mesure ($M'_Z$ , $M'_A$ , $M'_W$) obtenus au moyen du rayonnement infrarouge et représentant les grammages de la matière-support (Z), du premier composant de charge (A) et de l'autre corps (W) et d'un signal de mesure théorique ($M'_B$) représentant le grammage du deuxième composant de charge (B), le signal de mesure théorique ($M'_B$) étant déterminé en se servant en plus du signal de mesure ($E_T$) obtenu au moyen du rayonnement X caractéristique et représentant l'extinction totale de la bande de matériau en prenant comme hypothèse que le signal de mesure théorique ($M'_B$) représentant le grammage du deuxième composant de charge (B) est faussé de la même manière que le signal de mesure ($M'_A$) obtenu au moyen du rayonnement infrarouge et représentant le grammage du premier composant de charge (A).

2. Procédé selon la revendication 1, caractérisé en ce que pour déterminer une valeur de référence corrigée pour les intensités de rayonnement mesurées avec les longueurs d'onde de mesure du rayonnement infrarouge, on utilise deux longueurs d'onde de comparaison ($\lambda_1$, $\lambda_2$) qui ne sont pour l'essentiel pas absorbées par les matières de la bande de matériau.

3. Procédé selon l'une des revendications 1 ou 2, caractérisé en ce que dans le cas où le premier composant de charge est constitué par les deux charges kaolin et talc, on utilise pour chacune de ces deux charges une longueur d'onde de mesure ($\lambda_{A1}$, $\lambda_{A2}$) absorbée par ladite charge dans la zone du rayonnement infrarouge.

4. Dispositif de mesure pour l'exécution du procédé selon l'une des revendications précédentes, caractérisé par une combinaison d'un dispositif de mesure par rayonnement infrarouge à longueurs d'ondes multiples (10 à 18), un dispositif de mesure par rayonnement bêta (20, 22), un dispositif de mesure par rayonnement X (30, 32) avec une source de rayonnement Fe-55 ainsi qu'un micro-ordinateur (50) pour l'interprétation des signaux de mesure obtenus par les trois dispositifs de mesure.

FIG. 1

ABSORPTIONS-
KOEFFIZIENT
[REL. EINH.]

100.0

KAOLIN

CaCO₃

10.0

Fe55

1.0

1.0  2.0  3.0  4.0  5.0  6.0

ENERGIE

[keV]

EP 0 272 645 B1

REL. INTENSITÄT

BREMS-
STRAHLUNG

F I G . 2

Fe 55

5.9   ENERGIE

[keV]

10
IR-
STRAHLUNGS-
QUELLE

20
BETA-
STRAHLUNGS-
QUELLE

30
Fe-55-
STRAHLUNGS-
QUELLE

16

14

18   M   16

40

12
IR-
STRAHLUNGS-
DETEKTOR

22
BETA-
STRAHLUNGS-
DETEKTOR

32
x-STRAHLUNGS-
DETEKTOR

M I K R O R E C H N E R   50

F I G . 4

13

F I G . 3

EP 0 272 645 B1

EP 0 272 645 B1

$$M_B' = M_B \frac{M_A'}{M_A} \qquad 56$$

$$M_A' = \frac{E_A'}{\alpha_A} \qquad 51$$

$$M_Z' = \frac{E_Z'}{\alpha_Z} \qquad 52$$

$$M_W' = \frac{E_W'}{\alpha_W} \qquad 53$$

$$M_A = M_A' \frac{M_T}{M_A' + M_B' + M_Z' + M_W'}$$

$$M_Z = M_Z' \frac{M_T}{M_A' + M_B' + M_Z' + M_W'}$$

$$M_W = M_W' \frac{M_T}{M_A' + M_B' + M_Z' + M_W'}$$

$$54$$

$$M_B = \frac{E_T - \mu_A M_A - \mu_Z M_Z - \mu_W M_W}{\mu_B} \qquad 55$$

F I G . 5